(19) Europäisches Patentamt / European Patent Office / Office européen des brevets

(11) **EP 3 593 793 B1**

## (12) EUROPEAN PATENT SPECIFICATION

(45) Date of publication and mention
of the grant of the patent:
**01.09.2021 Bulletin 2021/35**

(51) Int Cl.:
**A61K 9/51** (2006.01)

(21) Application number: **18183009.2**

(22) Date of filing: **11.07.2018**

(54) **ONE-POT FORMULATION OF BIOPOLYMER PARTICLES**

EINTOPFFORMULIERUNG VON BIOPOLYMERPARTIKELN

FORMULATION À POT UNIQUE DE PARTICULES DE BIOPOLYMÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**15.01.2020 Bulletin 2020/03**

(73) Proprietor: **Wanitcare GmbH**
**12437 Berlin (DE)**

(72) Inventors:
• **Bäumler, Hans**
**12437 Berlin (DE)**
• **Georgieva, Radostina**
**10119 Berlin (DE)**
• **Kloypan, Chiraphat**
**13353 Berlin (DE)**
• **Steffen, Axel**
**13086 Berlin (DE)**
• **Xiong, Yu**
**12249 Berlin (DE)**

(74) Representative: **Zimmermann & Partner**
**Patentanwälte mbB**
**Postfach 330 920**
**80069 München (DE)**

(56) References cited:
• **XIONG YU ET AL: "Structure and properties of hybrid biopolymer particles fabricated by co-precipitation cross-linking dissolution procedure", JOURNAL OF COLLOID AND INTERFACE SCIENCE, ACADEMIC PRESS,INC, US, vol. 514, 12 December 2017 (2017-12-12), pages 156-164, XP085344255, ISSN: 0021-9797, DOI: 10.1016/J.JCIS.2017.12.030**
• **DMITRY V. VOLODKIN ET AL: "One-Step Formulation of Protein Microparticles with Tailored Properties: Hard Templating at Soft Conditions", ADVANCED FUNCTIONAL MATERIALS, vol. 22, no. 9, 9 May 2012 (2012-05-09), pages 1914-1922, XP55537147, DE ISSN: 1616-301X, DOI: 10.1002/adfm.201103007**
• **YU XIONG ET AL: "Hemoglobin-Based Oxygen Carrier Microparticles: Synthesis, Properties, and In Vitro and In Vivo Investigations", BIOMACROMOLECULES, vol. 13, no. 10, 8 October 2012 (2012-10-08), pages 3292-3300, XP55418512, US ISSN: 1525-7797, DOI: 10.1021/bm301085x**
• **CHIRAPHAT KLOYPAN ET AL: "Improved oxygen storage capacity of haemoglobin submicron particles by one-pot formulation", ARTIFICIAL CELLS, NANOMEDICINE AND BIOTECHNOLOGY, 2 November 2018 (2018-11-02), pages 1-9, XP055531330, US ISSN: 2169-1401, DOI: 10.1080/21691401.2018.1521819**

Remarks:
The file contains technical information submitted after the application was filed and not included in this specification

**Description**

TECHNICAL FIELD

Embodiments of the present invention relate to the fabrication of biocompatible particles, in particular to protein particles comprising cross-linked biofunctional proteins as well as particles formed by other biofunctional polymers, such as peptides, aminocarbohydrates, and nucleic acids.

BACKGROUND

In the last decades, protein particles have been proposed for a number of biomedical applications such as drug delivery systems and hemoglobin-based oxygen carriers. Many techniques are available for the fabrication of protein nano- and micro particles including spray drying, emulsification, layer-by-layer assembly, solvent displacement and polymersome formation. However, each of these techniques has serious drawbacks which impair the function and/or biocompatibility of the obtained nano- and microparticles. For example, spray drying or lyophilization techniques deliver protein particles in dry powder formulation but usually their polydispersity is high. On the other hand entrapment efficiency is usually poor in protein particles prepared by solvent displacement. Apart from that, tertiary and quaternary structures of proteins need to be preserved to maintain protein function which is hard to fulfill by these conventional methods due to exposure to high temperature, high ionic strength, non-physiological pH, organic solvents or hydrophobic interfaces. D.V. Volodkin, S. Schmidt, P. Fernandes, N.I. Larionova, G.B. Sukhorukov, C. Duschl, H. Möhwald, and R. von Klitzing describe in Advanced Functional Materials 2012, 22, 1914-1922 a one-step formulation of protein microparticles with tailored properties by hard templating at soft conditions.

Previously, we have described a method for preparing micro- and submicro-particles called coprecipitation-crosslinking-dissolution (CCD) (Yu Xiong, Radostina Georgieva, Axel Steffen, Kathrin Smuda, Hans Bäumler; Journal of Colloid and Interface Science 2018, 514, 156-164; Yu Xiong, Axel Steffen, Kristin Andreas, Susanne Müller, Nadine Sternberg; Radostina Georgieva, and Hans Bäumler; Biomacromolecules 2012, 13, 3292-3300), which consists of three main steps: A protein and an inorganic template are precipitated together followed by a crosslinking step where glutaraldehyde is used to crosslink the precipitated protein molecules. Finally, EDTA is used to selectively remove the inorganic template to obtain protein microparticles. This technique can be used to fabricate in mild conditions pure protein particles with uniform morphology and narrow size distribution. However, the crosslinking of proteins with glutaraldehyde has certain drawbacks.

Glutaraldehyde (GA) can react with several functional groups of proteins, such as amine, thiol, phenol, and imidazole and is very effective as a crosslinker of proteins because the most reactive amino acid side-chains are nucleophiles. However, there are many concerns relating to undesired properties of glutaraldehyde crosslinked biomaterials such as autofluorescence as well as its toxicity.

SUMMARY

Against this background, according to an embodiment, a method for preparation of biopolymer particles is suggested, the method comprising:

- fabricating an aqueous suspension of precipitate particles by mixing together a first salt, a second salt, a biopolymer and an oxidized carbohydrate having a molecular weight of 10 kDa or more,
  wherein the oxidized carbohydrate is selected from an oxidized dextran, an oxidized hydroxyethyl starch, an oxidized hyaluronic acid, an oxidized alginate, an oxidized chitosan, an oxidized chondroitin, an oxidized pectin, and an oxidized cellulose,
  wherein the biopolymer is selected from the group consisting of: proteins originated from plants, algae or animals, amino-carbohydrates originated from plants, algae or animals, peptides originated from plants, algae or animals, and nucleic acids originated from plants, algae or animals,
  wherein the first and the second salt have anions of inorganic nature, wherein the first and the second salt are selected such that the anion of the first salt and the cation of the second salt together form precipitate particles, wherein upon mixing the biopolymer and the carbohydrate are incorporated in the precipitate particles and cross-link with each other;

- washing the suspension of precipitate particles;

- dissolving the precipitate particles by adding a chelator and releasing the cross-linked biopolymer and the carbohydrate as biopolymer particles.

Further, according to an embodiment, a biopolymer particle is suggested, the biopolymer particle comprising a biopolymer which is cross-linked by a carbohydrate, wherein the biopolymer and the carbohydrate form together a 3-dimensional network extending through the volume of the particle, wherein the carbohydrate has a molecular weight of 10 kDa or more, and the carbohydrate is selected from dextran, hydroxyethyl starch, hyaluronic acid, alginate, chitosan, chondroitin, pectin, and cellulose, wherein the biopolymer is selected from the group consisting of: proteins originated from plants, algae or animals, amino-carbohydrates originated from plants, algae or animals, peptides originated from plants, algae or animals, and nucleic acids originated from plants, algae or animals, and wherein the 3-dimensional network has a molecular weight of at least 1 MDa.

Furthermore, according to an embodiment, a kit comprising biopolymer particles is suggested, wherein the kit comprises: a dry powder of biopolymer particles which are produced as suggested, said biopolymer particles comprising cross-linked molecules selected from the group consisting of: proteins originated from plants, algae or animals; amino-carbohydrates originated from plants, algae or animals; peptides originated from plants, algae or animals; and nucleic acids originated from plants, algae or animals; and a physiological solution.

Furthermore, according to an embodiment a use of an oxidized carbohydrate having a molecular weight of at least 10 kDa to form biopolymer particles is suggested, wherein the biopolymer particles comprise a three-dimensional network structure extending through the volume of the particle, wherein the three-dimensional network structure is formed at least by a biopolymer and a carbohydrate, the carbohydrate cross-linking the biopolymer, wherein the carbohydrate has a molecular weight of 10 kDa or more and wherein the oxidized carbohydrate is selected from an oxidized dextran, an oxidized hydroxyethyl starch, an oxidized hyaluronic acid, an oxidized alginate, an oxidized chitosan, an oxidized chondroitin, an oxidized pectin, and an oxidized cellulose, and wherein the three-dimensional network has a molecular weight of at least 1 MDa.

Further details and features of suggested embodiments are provided with the following description and claims, and further illustrated by the attached figures.

BRIEF DESCRIPTION OF THE DRAWINGS

The components in the figures are not necessarily to scale, instead emphasis being placed upon illustrating the principles of the invention. In the drawings:

**Figure 1** illustrates schematically the particle preparation through the one-pot fabrication technique.

**Figure 2A-2D** are scanning electron micrographs of (A) 40T-APs, (B) 70T-APs, (C) 40T-HbMPs, and (D) 70T-HbMPs. Therein 40T stands for Odex with a molecular weight of 40 kDa, 70 T stands for Odex with a molecular weight of 70 kDa. Hb stands for hemoglobin, HbMPs stands for hemoglobin particles, and APs stands for albumin particles.

**Figure 3** is a graph showing the compression and dilation behavior of HbMPs and APs.

**Figure 4** shows the absorption spectra of hemoglobin (Hb), hemoglobin particles (HbMPs), and albumin particles (APs).

**Figure 5** shows flow cytometry results (side scatter (SSC) vs. forward scatter (FSC) dot plot) of APs crosslinked with Odex (Odex-APs) and glutaraldehyde (GA-APs), respectively. The Odex-APs show no detectable autofluorescence in contrast to the GA-APs (fluorescence channels FITC-A and PE-A).

**Figure 6** is a dot plot FITC/FSC which demonstrates phagocytic activity of leukocyte and leukocyte engulfed FITC-labelled E. coli after pre-incubation with E. coli (positive control), PBS (negative control), and 40T-HbMPs.

**Figure 7** is a graph showing the percentage of phagocytic activity of granulocyte engulfed FITC-labelled E. coli (positive control), PBS (negative control), where APs and HbMPs were pre-fed to granulocytes followed by a second feeding step with FITC-labelled E. coli (detective bacteria).

**Figure 8** shows dot plots depicting the activation of platelets after pre-incubation of 70T-HbMPs

**Figure 9** is a chart illustrating the effect of APs and HbMPs on arachidonic acid (AA)-, collagen-, and epinephrine (Epi)- induced platelet activation.

**Figure 10** is a plot illustrating the preservation of functional activity of hemoglobin depending on the used cross-linker.

DETAILED DESCRIPTION

In the following detailed description, reference is made to the accompanying drawings, which form a part hereof, and in which are shown by way of illustration of specific embodiments in which the invention may be practiced. The following detailed description, therefore, is not to be taken in a limiting sense, and the scope of the present invention is defined by the appended claims. The foregoing and other objects, features, and advantages of the invention will become more apparent from the following detailed description, which proceeds with reference to the accompanying figures.

Although specific terms are used herein for the sake of clarity, these terms are intended to refer only to the particular structure of the embodiments selected for illustration in the drawings, and are not intended to define or limit the scope of the disclosure.

Numerical values in the specification and claims of this application should be understood to include numerical values which are the same when reduced to the same number of significant figures and numerical values which differ from the stated value by less than the experimental error of conventional measurement technique of the type described in the present application to determine the value.

All ranges disclosed herein are inclusive of the recited endpoint and independently combinable. For example, the range of "from 5 nm to 100 $\mu$m" is inclusive of the endpoints 5 nm and 100 $\mu$m, and all the intermediate values. The endpoints of the ranges and any values disclosed herein are not limited to the precise range or value; they are sufficiently imprecise to include values approximating these ranges and/or values.

As used herein, approximating language may be applied to modify any quantitative representation that may vary without resulting in a change in the basic function to which it is related. Accordingly, a value modified by a term or terms, such as "about" and "substantially," may not be limited to the precise value specified, in some cases. In at least some instances, the approximating language may correspond to the precision of an instrument for measuring the value. The modifier "about" should also be considered as disclosing the range defined by the absolute values of the two endpoints. For example, the expression "from about 2 to about 4" also discloses the range "from 2 to 4." Furthermore, throughout this application, the term "about" is used to indicate that a value includes the standard deviation of error for the device or method being employed to determine the value.

Other embodiments of the invention are discussed throughout this application. Any embodiment discussed with respect to one aspect applies to other aspects as well and vice versa. Each embodiment described herein is understood to be embodiments that are applicable to all aspects of the invention. It is contemplated that any embodiment discussed herein can be implemented with respect to any device, method, or composition, and vice versa. Furthermore, systems, compositions, and kits of the invention can be used to achieve methods of the invention.

The use of the word "a" or "an" when used in conjunction with the term "comprising" in the claims and/or the specification may mean "one," but it is also consistent with the meaning of "one or more," "at least one," and "one or more than one."

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claims, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

As used in this description (above and below) and claims, the used word "about" before a numerical value indicates a range of numerical values encompassing, i.e. including, a statistical deviation of $\pm$ 5% of the indicated numerical value.

The terms "particle" and "particles" refer to particles having a diameter of 1000 micrometers ($\mu$m) or less. In particular, the term "particles" is intended to include microparticles and nanoparticles, wherein microparticles are particles having a diameter ranging from 1 $\mu$m to 1000 $\mu$m, and nanoparticles are particles having a diameter ranging from 1 nanometer to 1000 nanometers. The particles will be referred to in this disclosure as being spherical, though perfect sphericity is not required and other shapes, e.g. peanut-like shapes, rhombohedral or star-like shapes are acceptable. As used herein, the indicated particle size relates to values detected by atomic force microscopy (see below). If hydrodynamic particle diameters are indicated, they have been detected by dynamic light scattering, e.g. with a Zetasizer (Malvern Instruments Ltd. / Malvern Panalytical). For non-spherical particle geometries observed e.g. by atomic force microscopy (see below) an average value calculated as the arithmetic mean value of the shortest and the longest diameter can be used to express the median particles size.

Polysaccharides, i.e. carbohydrates, are natural biopolymers which have a great number of reactive groups on molecular chains, such as hydroxyl, carboxyl, and amino groups which can make them be easily modified chemically and biochemically. Polysaccharides are highly safe, non-toxic, biodegradable, and biocompatible. Besides, polysaccharides have abundant resources in nature and low cost in their processing. They are available from various resources including animals (e.g. chitosan, chondroitin), plants (e.g. pectin, cellulose), algae (e.g. alginate), and microorganisms (e.g. dextran). The oxydized carbohydrate which is used for cross-linking of at least two biopolymer molecules has preferably no aminogroups. Advantageously, an intramolecular cross-linking of the oxidized carbohydrate can thus be prevented.

Dextran is a homopolymer of glucose, produced , e.g., by the bacteria called Leuconostoc mesenteroides. Dextrans consist essentially of $\alpha$-1,6 linked D-glucopyranose residues with a few percent of $\alpha$-1,2, $\alpha$-1,3, or $\alpha$-1,4-linked side chains. Dextran is selected in many biomedical applications because it is slowly degraded by human enzymes compared to other polysaccharides (e.g. glycogen with $\alpha$-1,4 linkages) and cleaved by microbial dextranases in the gastrointestinal tract. It has been used as a macromolecular carrier for the delivery of drugs and proteins, primarily to increase the durability of therapeutic agents in the blood circulation. Furthermore, dextran has been used to decrease the *in vivo* immunogenicity of proteins and enzymes. Dextran has been selected as a bio-crosslinker because of its chemical properties such as high water-solubility, high stability, high content of hydroxyl groups, which are suitable for derivatization. Further, the used therein oxidation of dextran by periodate ions is a common dextran functionalizing modification reaction which yields a purified product by using a simple dialysis method. The glucose residue of dextran has vicinal diol which houses two oxidizable bonds. The oxidation of those bonds forms an aldehyde in $C_3$, which is susceptible to periodate oxidation on account of the presence of the hydroxyl groups in $C_2$ and $C_4$ and subsequently leads to a double oxidation of the same residue. This leads to opening of the glucose ring and formation of double aldehyde groups, which may react with amino groups creating a Schiff base (Maillard reaction). The reaction may proceed under mild conditions with high efficiency and does not require any catalyst. To the best of our knowledge, oxidized dextran (Odex) was previously not used for fabrication of particles such as micro- or nano- protein particles.

The present disclosure provides a novel particle fabrication method based on the CCD technique by applying an oxidized carbohydrate, e.g. an oxidized dextran (Odex) as a cross-linker for bio-polymers e.g., for polyelectrolytes, amphiphiles, in particular for proteins such as albumin, and hemoglobin. Here, the co-precipitation and the cross-linking steps are combined in one single step because the cross-linker Odex is co-precipitated together with the biopolymers. Advantageously, using an oxidized carbohydrtae for cross-linking allows instantaneous cross-linking. That means that no extra cross-linking step is required. Thus, the particle fabrication is simplified and can be carried out as a one-pot process. Furthermore, the cross-links within the three-dimensional network of the particle are distributed homogeneously. That results in a higher stability of the particles. Several physiochemical properties of the resulting particles were investigated including size, zeta-potential, spectrophotometric absorption. Further, scanning electron microscopy imaging was used to characterize the morphology of the produced particles. Additionally, the influence of these novel particles on phagocytosis activities of human granulocyte and monocyte as well as platelet activation were measured *in vitro,* as important factors concerning biocompatibility.

In specific embodiments, the resulting protein particles, e.g. particles generated from proteins which are cross-linked by an oxidized carbohydrate, retain at least 40% of their original specific activity. Preservation of an enzyme activity of the protein as high as 90 % can be reached. For hemoglobin a preservation of the specific biological activity (oxygen transport) as high as 40 % could be reached. Typically, the particles comprise 40% through 85 % (w/w) protein and a carbohydrate content of the particles is correspondingly 60% (w/w) through 15 % (w/w).

According to preferred embodiments the arithmetic mean diameter of the biopolymer particles as generated by synchronous coprecipitation and cross-linking with an oxidized carbohydrate are between 500 nm and 10 μm, typically between 500 nm and 5 μm, for instance between 500 nm and 1 μm. The particles comprise at least two biopolymers, wherein one of the biopolymers is the cross-linker.

Other objects, features and advantages of the present invention will become apparent from the following detailed description. It should be understood, however, that the detailed description and the specific examples, while indicating specific embodiments of the invention, are given by way of illustration. With this background in mind, the methods disclosed herein will be described in more detail below.

Thus, according to an embodiment, the invention provides a method of manufacturing biopolymer particles, the method comprising:

- fabricating an aqueous suspension of precipitate particles by mixing together a first salt, a second salt, a biopolymer and an oxidized carbohydrate having a molecular weight of 10 kDa or more,

   wherein the oxidized carbohydrate is selected from an oxidized dextran, an oxidized hydroxyethyl starch, an oxidized hyaluronic acid, an oxidized alginate, an oxidized chitosan, an oxidized chondroitin, an oxidized pectin, and an oxidized cellulose,
   wherein the biopolymer is selected from the group consisting of: proteins originated from plants, algae or animals, amino-carbohydrates originated from plants, algae or animals, peptides originated from plants, algae or animals, and nucleic acids originated from plants, algae or animals,
   wherein the first and the second salt have anions and cations of inorganic nature, wherein the first and the second salt are selected such that the anion of the first salt and the cation of the second salt together form a template structure of the precipitate particles,
   wherein upon mixing the biopolymer and the carbohydrate are incorporated in the template structure of the precipitate particles and cross-link with each other;

- washing the suspension of precipitate particles; and

- dissolving the template structure of the precipitate particles by adding a chelator and releasing the cross-linked biopolymer and the carbohydrate as biopolymer particles.

Therein, at least one of the first salt, the second salt, the biopolymer and the oxidized carbohydrate is in a solubilized state before being mixed together in one pot. Typically, at least the first salt and the second salt are provided separately. However, both salts can be provided together in a dry state as well, e.g. as a crystalline powder.

Further, the first and the second salt are selected such that the anion of the first salt and the cation of the second salt together form an insoluble precipitate. During the mixing the forming precipitate serves as a solid substrate, i.e. a template, for the adsorption of the biopolymer which is instantly cross-linked by the oxidized carbohydrate. The mixing is accomplished by vigorous stirring. After washing the precipitates, e.g. comprising a filtration or centrifugation step, the precipitates are dissolved and the biopolymer particles are released. Typically, the anions are of inorganic nature and can be selected from the group containing Ca-ions, Mn-ions, Mg-ions, Ba-ions and mixtures thereof. Further, the cations are typically of inorganic nature and can be selected from the group containing carbonate-ions, phosphate-ions, hydrogen phosphate-ions, dehydrogen phosphate-ions, and mixtures thereof.

For example, the first salt may comprise $CaCl_2$ or $MnCl_2$ for providing Ca-ions or Mn-ions, respectively. The second solution may comprise $Na_2CO_3$ for providing carbonate-ions. In the precipitation step $CaCO_3$ or $MnCO_3$ precipitates are formed. A skilled person will appreciate that the cations and anions are not restricted to the above examples. Other examples of template structures formed by the respective ions include, without being limited thereto, all calcium phosphate particles, such as $Ca_3(PO_4)_2$, $CaHPO_4$ and $Ca(H_2PO_4)_2$, and $BaCO_3$. The suggested method of manufacturing is time-saving and cost effective. In comparison to the previously known CCD technique the previously separate cross-linking step is now combined with the step of co-precipitation of protein and porous template. In other words, the protein which adsorbs on the precipitating template is synchronously cross-linked by the oxidized carbohydrate. Thus, the new method is less laborious. The suggested new technique allows to entrap about 70% of the protein which is present in the starting solution in typically spherically or peanut-like shaped particles.

According to an embodiment, the dissolving step comprises adding to a suspension of precipitates a chelator, wherein the chelator is selected from the group consisting of: diethylenetriamine pentaacetic acid, ethylenediamine tetracetic acid (EDTA), N-hydroxyethylethylenediamine triacetic acid, nitrilo triacetic acid, and salts thereof.

Advantageously, these chelators are commercially available and approved for use, as e.g. EDTA in medical applications, cosmetics or food industry.

According to an embodiment in the fabricating step the first salt and the second salt are provided in separate solutions, and wherein the biopolymer and the oxidized carbohydrate are provided in separate solutions.

Advantageously, providing the salts in a solute state ready to be mixed allows for immediate generation of the precipitate. If combined with a suitable stirring speed, e.g. at 600 rpm (rotation per minute), the size of produced particles can be controlled. Typically, stirring speeds of 200-600 rpm at room temperature, typically 600 rpm as specified in Xiong, Y.; Georgieva, R.; Steffen, A.; Smuda, K.; Bäumler, H. (2018) "Structure and properties of hybrid biopolymer particles fabricated by co-precipitation cross-linking dissolution procedure" (Journal of Colloid and Interface Science 514: 156-164) can be used.

According to an embodiment, the oxidized carbohydrate comprises a linear or a branched carbohydrate.

Dextran consists of glucose subunits, which are linked via the $\alpha$-1,6 glucose units, which gives dextran a high flexibility. Branched molecules such as hydroxyethyl starch (HAES) are less suitable because their mobility in the template is limited. Advantageously linear carbohydrates allow effective cross-linking of biopolymers, even if their molecular weight is comparable low.

According to an embodiment, the oxidized carbohydrate comprises an oxidized dextran.

Advantageously, dextran is allowed in medical applications and can be used, e.g. as a blood substitute, i.e. volume expander or plasma substitute. Dextran 70, i.e. a dextran having molecular weight of 70 kDa, is on the WHO Model List of Essential Medicines. Oxidized polysaccharides such as, e.g. oxidized dextrans offer certain advantages over previously known cross-linker, in particular over glutaraldehyde. Advantageously, undesirable risks and side-effects of the previously used glutaraldehyde (GA) can be omitted by using the new cross-linking agent. Furthermore, laborious blocking and washing procedures can be dispensed with.

A comparison of the obtained particle's properties (advantages and disadvantages) is compiled in the table below.

|  | Advantage | Disadvantage |
|---|---|---|
| Glutaradehyde (GA) | Very reactive, because of its two reactive aldehyde moieties which can react with a biopolymer.<br><br>Commercially available at low cost. | Limited in use for intermolecular protein cross-linking due to small size compared to Odex. The cross-linker size and the distance between groups to be cross-linked should be similar.<br>Crosslinking of polymers is partly reversible, which leads to a leakage of polymers out of the particles.<br>Has been related to adverse health and environmental effects by Huang YR, Hung YC, Hsu SY, Huang YW, Hwang DF (2008) Application of electrolyzed water in the food industry. Food Control 19:329-345, and by Zeiger E, Gollapudi B, and Spencer P. (2005) Genetic toxicity and carcinogenicity studies of glutaraldehyde - a review. Mutation Research/Reviews in Mutation Research 589(2): 136-151 |
| Oxidized dextran (Odex) | Easily prepared under mild conditions;<br>Periodate-oxidized dextran is considered to be a very reactive polyaldehyde compound towards N-nucleophiles, including amines and hydrazines;<br>Lattice formation of Odex and protein is faster than GA as a result of a bigger molecule of Odex.<br>In comparison with CCD the amount of functional hemoglobin is higher (CCD 30%, OdexHb 40%) and this value is stable during storage for OdexHb, but not for CCD, where the functional Hb decreases rapidly with storage time.<br>Results in rapidly setting biopolymer-based material without any extraneous toxic cross-linking agents, and is rather simple, inexpensive, non-toxic, biocompatible, and biodegradable Can be used in a variety of clinical situations.<br>No leakage of crosslinked polymers out of the particles stored in salt solution for at least 6 months. | Not available commercially yet, but this is no real disadvantage. |

According to an embodiment the biopolymer particles have a D90 diameter of 10 $\mu$m or less, particularly of 5 $\mu$m or less, more particularly of 2 $\mu$m or less, wherein the value D90 describes the diameter where ninety percent of the distribution has a smaller particle size than the indicated value or a particle size equal to the indicated value and ten percent has a larger particle size than the indicated value.

Advantageously, particles of such diameters are easily transported in the blood stream of a living organism. Furthermore, these particles remain sufficiently long in circulation in order to fulfill the designed function, e.g. oxygen delivery/gas exchange, drug release or targeted drug release. In contrast to many previously known nanoparticles, without a previous surface modification, these particles once injected intravenously, will not leave the vessels and will not pass barriers (like the blood-brain-barrier /BBB or skin barrier) in an uncontrolled manner.

According to an embodiment the suggested method further comprises fractionizing the biopolymer particles with respect to size in order to separate particles having a size of 500 nm or less from the remaining particles.

The mentioned separation can be reached, e.g. by centrifugation or crossflow filtration. These smaller particles are suitable after surface modification to deliver drugs through the BBB.

As indicated above, the biopolymer is selected from the group consisting of: proteins originated from plants, algae or animals, amino-carbohydrates originated from plants, algae or animals, peptides originated from plants, algae or animals, and nucleic acids originated from plants, algae or animals.

Advantageously, all these biomolecules can be cross-linked by the oxidized carbohydrate as selected. In particular, proteins from animal origin can be selected among albumins, serum proteins, metallo-proteins, enzymes and antibodies. Examples of preferred metallo-proteins are hemoglobin and myoglobin. Another protein used for particle formation is silk protein. General examples of preferred enzymes are metalloproteases or such which catalyze reactions such as phosphorylation/dephosphorylation, carboxylation/decarboxylation and acetylation. Examples of preferred proteins of plant origin are, e.g. soy-proteins, plant seed proteins and lectins (e.g. ConA).

According to an embodiment, if an oxidized dextran was selected to serve as the cross-linker, the dextran is selected to have a molecular weight of between 10 kDa and 2 MDa, in particular a molecular weight of between 20 kDa and 1 MDa, e.g. of between 30 kDa and 400 kDa, and more particularly a molecular weight of between 40 kDa and 100 kDa. According to typical embodiments, dextrans of different molecular weight can be oxidized in order to serve as a suitable cross-linking agent. For instance, a dextran of M.W. 10,000 Da, a dextran of 40 kDa, a dextran of 70 kDa, a dextran of about 100 kDa, dextrans of larger M.W., even a dextran of a M.W. of about 500 kDa or 2 MDa can be oxidized, e.g., by sodium periodate, in order to obtain the suitable oxidized dextran (Odex) for cross-linking.

An oxidized dextran of high molecular weight offers a large number of possible cross-linking groups, whereas oxidized dextrans of lower molecular weight provide less cross-linking groups. Advantageously, the number of possible cross-linking groups can be adjusted via concentration and/or molecular weight of oxidized dextran (in contrast to glutaraldehyde)

According to an embodiment, a biopolymer particle comprising a 3-dimensional network structure is suggested. The biopolymer particle comprises a biopolymer which is cross-linked by a carbohydrate. The biopolymer forms together with the carbohydrate a 3-dimensional network which extends through the volume of the particle, wherein the carbohydrate has a molecular weight of 10 kDa or more and the carbohydrate is selected from dextran, hydroxyethyl starch, hyaluronic acid, alginate, chitosan, chondroitin, pectin, and cellulose, wherein the biopolymer is selected from the group consisting of: proteins originated from plants, algae or animals, amino-carbohydrates originated from plants, algae or animals, peptides originated from plants, algae or animals, and nucleic acids originated from plants, algae or animals, and wherein the 3-dimensional network has a molecular weight of at least 1 MDa, for instance of at least 5 MDa, typically of at least 10 MDa.

Advantageously, the cross-linking by the carbohydrate preserves the specific biological activity of the biopolymer. In particular, the three-dimensional structure of the biopolymer molecules comprising the particle is preserved. For instance substrate-binding pockets of an enzyme, epitope recognizing structures of an antibody or oxygen-binding sites of a heme-protein are prevented from damage and allow fulfilling of the designated function by the particle. Further, due to their 3-dimensional network structure the particles are resilient and thus are easily transported even through thin capillaries. Preferably, the suggested biopolymer particles comprise at least one biologically active protein which is partially cross-linked by a bio-compatible cross-linking agent, particularly by an oxidized carbohydrate. The particle is free from any glutaraldehyde. Preferred proteins are selected from, e.g. metallo-proteins, in particular from hemoglobin and myoglobin. Other preferred proteins may be selected from serum proteins, particularly from albumin, e.g. human serum albumin. Different types of proteins may be present in one particle as well. Within the particle the different proteins can be interconnected with each other for example proteases and proteins. It is noted that the molecular weight of the oxidized dextran (odex) may favorably be chosen depending on the number of binding sites ("pockets") on the biopolymer, which shall be cross-linked: The higher the molecular weight of the oxidized dextran the lower will be the amount of intramolecular cross-links and the higher will be the degree of preserved biological activity, because a high degree of intramolecular cross-links results in reducing the native biological activity. Whereas glutaraldehyde generates both intra- and inter-molecular cross-links, oxidized carbohydrates such as Odex generate mainly intermolecular cross-links. Therefore, advantageously the molecules of the chosen biopolymer are cross-linked with each other.

According to an embodiment, the invention provides biopolymer particles containing about 60%-97% (w/w) of a protein, e.g. albumin or hemoglobin, and about 3%-40% (w/w) of a carbohydrate, wherein a backbone of the carbohydrate crosslinks sections of the protein chain(s). Depending on the desired amount of polymer in the particle, a range between 40 and 85% (w/w) of the protein could be used as well. Accordingly, 15-60% (w/w) of an oxidized carbohydrate can be used for crosslinking. The carbohydrate backbone is typically characterized by at least partially repeating units.

Advantageously, varying the amount of the cross-linking carbohydrate within the particles allows adapting a hydrophobicity index of the artificial particle. Even strongly hydrophobic proteins which otherwise might tend towards aggregation in an aqueous environment yield stable particle suspensions free of particle aggregates. Furthermore, by selecting an appropriate molecular weight, a favorably high ratio of inter- to intra-molecular cross-links can be chosen.

According to an embodiment, the suggested particles comprise a metallo protein, in particular hemoglobin or myoglobin, and the biopolymer particle is able to bind and to release, depending on a partial gas pressure, both oxygen and carbon dioxide.

Advantageously such particles can be used as a blood substitute.

According to an embodiment, the suggested particles are suspended in a physiological solution, in particular in a 0.9 % aqueous NaCl solution, in a Ringer's salt solution, or in a serum.

Advantageously, such suspension of particles is biocompatible and can be injected into the bloodstream of a living organism.

According to an embodiment, the suggested particle further comprises a releasable drug, wherein the releasable drug is bound to the 3-dimensional network, e.g. by a link which can be specifically cleaved at the target site, e.g. by an enzyme. Further, the drug may be bound by an ionic bond and thus be releasable, e.g. by a change of the pH-value at the target site (tissue). The drug may also be bound by hydrophobic interaction with the 3-dimensional network structure, e.g. with the biopolymer of the particle or can be simply adsorbed to it. Furthermore, hydrolysis can be used for release of the drug. Such hydrolysis can be triggered, e.g. by a focused electromagnetic energy or ultra sound the target site is exposed to.

Advantageously, such particles can be used for targeted drug delivery. The drug can be released continuously while the particle circulates in the blood stream, or can be released preferentially after the particle has been taken up by a target tissue.

According to an embodiment, the surface of the suggested particle is functionalized, i.e. decorated with functional groups, with peptides, or sialic acid derivatives which allow the particle be delivered to a target tissue.

Advantageously, a drug which is carried by the particle can thus be released at the target tissue. Unwanted side-effects of the drug can thus be minimized.

According to an embodiment, the invention provides a kit, the kit comprising dry biopolymer particles and a physiological solution. The dry biopolymer particles comprise cross-linked protein like hemoglobin, myoglobin, serum albumin, collagen or mixtures thereof. The physiological solution comprises, e.g., a 0.9 % NaCl solution; a Ringer's salt solution, or a buffered saline. A Ringer's salt solution may comprise, e.g., per 1 ml: 0.147 mmol $Na^+$; 4 $\mu$mol $K^+$; 2.25 $\mu$mol $Ca^{2+}$; and 0.156 mmol $Cl^-$. Other additives contained in the Ringer's salt solution may comprise, e.g., $Mg^{2+}$, lactate, acetate or glucose.

Advantageously, the kit can be easily stored, and the particles be prepared immediately before use. Thus, particle suspensions can be provided even in regions suffering under insufficient infrastructure. Sterility of the particles can be preserved for a long time even at higher storage temperatures. Furthermore, the quantity and concentration of particles in a suspension can easily be varied depending on the current requirements.

According to an embodiment, the particles of the kit comprise a cross-linked protein of animal origin, more particularly a hemoglobin and/or an albumin.

Advantageously, hemoglobin particles prepared by the specified method may serve as oxygen carriers. They can, at least temporarily, fulfill the function of natural red blood cells (erythrocytes). The provided biopolymer particles are able to bind and to release, *inter alia* depending on a partial gas pressure, both oxygen and carbon dioxide, respectively. Particles comprising albumin can favorably serve as drug carriers since albumin is able to adsorb and release different chemical substances which are pharmaceutically active.

According to an embodiment, the particles comprise a serum protein, preferably human serum albumin.

Advantageously from a production point of view, the size of the particles can be controlled by addition of serum albumin into the initial solution. From an application point of view, the circulation time of particles comprising human serum albumin can favorably be enhanced.

According to an embodiment, the invention provides a suspension of biopolymer particles in a physiological solution, e.g. suspended in about 0.9% aqueous NaCl or in a Ringer's salt solution. Such a suspension can be transfused directly into the blood system of a recipient as a blood substitute. Advantageously, the blood group of the recipient is not important and does not need to be considered.

According to an embodiment an use of an oxidized carbohydrate having a molecular weight of at least 10 kDa to form biopolymer particles is suggested, wherein the biopolymer particles comprises a 3-dimensional network structure extending through the volume of the particle, wherein the 3-dimensional network structure is formed at least by a biopolymer and a carbohydrate, the carbohydrate cross-linking the biopolymer, wherein the carbohydrate is selected from a dextran, a hydroxyethyl starch, a hyaluronic acid, an alginate, a chitosan, a chondroitin, a pectin, and a cellulose, wherein the carbohydrate has a molecular weight of 10 kDa or more and wherein the oxidized carbohydrate is selected from an oxidized dextran, an oxidized hydroxyethyl starch, an oxidized hyaluronic acid, an oxidized alginate, an oxidized chitosan, an oxidized chondroitin, an oxidized pectin, and an oxidized cellulose, and wherein the 3-dimensional network has a molecular weight of at least 1 MDa, for instance of at least 5 MDa, specifically of at least 10 MDa.

To summarize, the previously known coprecipitation-crosslinking-dissolution (CCD) technique for biopolymer microparticle fabrication was improved by omitting one preparation step by using carbohydrate cross-linker molecules, e.g. oxidized-dextran (Odex) or other oxidized carbohydrates. The co-precipitation and crosslinking were combined in one single step by combining incorporation of the cross-linker into the pre-particles with the gradual adsorption of proteins on the precipitates. According to one aspect, dextran (M.W. 40 and 70 kDa) was oxidized by sodium periodate to obtain Odex. Particles comprising human serum albumin (HSA) and/or bovine hemoglobin (Hb) were fabricated and characterized. The results demonstrate that periodate-oxidized dextran can crosslink protein molecules adsorbed by a crystalline inorganic precipitate to form biopolymer particles very effectively. The novel procedure of fabrication provides, e.g., albumin particles (APs) and hemoglobin particle (HbMPs) with equal size of approximately 850 nm, e.g. of 750 nm $\pm$ 250 nm, uniform morphology and negative zeta-potential. Furthermore, any influence of albumin particles (Aps) and HbMPs on phagocytic activity of granulocyte and activation/aggregation of platelets could not be observed. The results suggest that the proposed biopolymer particle fabrication technique is very effective and minimizes the fabrication time of biocompatible biopolymer particles.

EXAMPLES

Dextran (from *Leuconostoc mesenteroides,* M.W. 40 kDa and 70 kDa) was purchased from AppliChem GmbH, Germany. Human serum albumin (HSA) solution was purchased from Grifols GmbH, Germany. Ethylenediaminetetraacetic acid (EDTA), ferricyanide $K_3[Fe(CN)_6]$, glycine, manganese chloride, sodium borohydride, sodium carbonate, sodium chloride, sodium dithionite, sodium hydroxide, and sodium periodate were purchased from Sigma-Aldrich (Munich, Germany).

Hemoglobin (Hb) was extracted from bovine red blood cells by hypotonic hemolysis as previously described. Briefly, fresh bovine whole blood (obtained from Biophyll GmbH, Dietersburg, Germany, 2g/L EDTA-anticoagulated) was centrifuged at 2500 g for 15 min at 4 °C. The resulting packed red blood cells were washed three times with saline (0.9 % NaCl, B. Braun Melsungen AG, Germany). Hemolysis was performed by adding 5 volumes of lysing NaCl solution (100 mOsmol/kg). The solution was stirred at 4°C overnight and then centrifuged for 3 h at 5500 g. The supernatant was filtered by means of tangential flow filtration (TFF) using the KrosFlo® Research II TFF system (Spectrum Europe B.V., Netherlands) similar to the process described earlier. A 500 kDa mPES hollow fiber filter module (MiniKros Sampler, Spectrum) was used with a permeate flowrate of 30 to 25 mL/min at a transmembrane pressure (TMP) of 0.3 to 0.5 bar. The filtered hemoglobin was stored as a stock solution at -30 °C until use.

Dextran M.W. 40 and 70 kDa were oxidized by sodium periodate in a concentration 1:1 molar ratio of periodate:glucose subunit. Briefly, 6.6 g of sodium periodate (0.0309 mol) was added into 50 mL of 10% of dextran (0.0309 mol subunits) in water, shielded from light, with constant stirring at room temperature. The reaction was allowed to proceed for 1 hour. Then, the reaction mixture containing the oxidized dextran (Odex) was transferred to cellulose dialysis tube (cut off molecular weight of 12,000: Carl Roth GmbH, Germany) and dialyzed against a high volume of distilled water, which was changed every 12 h at 4°C for 3 days in dark conditions in order to separate the resulting salts and formic acid, which could alter the biocompatibility of the product. Afterward, the solution of oxidized dextran was degassed and stored under dark conditions at 4°C. Finally, the Odex solution was lyophilized by freeze-drying method and the obtained dried powder was maintained at room temperature for further usage.

The aldehyde group content of Odex was determined using the hydroxylamine hydrochloride method, unoxidized dextran samples were used as a control. 0.1 g of lyophilized 40T- and 70T-Odex were dissolved in 25 mL of 0.25 N hydroxylamine hydrochloride-methyl orange solution (pH 4.0). The mixtures were stirred on a magnetic stirrer for 24 hours and then titrated with standardized 0.1 N sodium hydroxide solution until the red-to-yellow endpoint was achieved by the equivalent of the color of the unoxidized-dextran titrated solution. Thereby, the number of aldehyde groups per 100 glucose subunits was calculated according to the following formula:

$$Aldehyde\ per\ 100\ glucose\ subunits = N \times V \times \left(\frac{MW}{wt}\right) \times \left(\frac{100}{S}\right)$$

where N is the concentration of standardized sodium hydroxide, *V* is the volume of NaOH solution used in the titration, *MW* is the molecular weight of dextran, *wt* is the weight of dextran, S is the number of glucose subunits in dextran.

Albumin particles (APs) or hemoglobin particles (HbMPs) were fabricated by a modified protocol based on the CCD technique previously described by Bäumler H, Xiong Y, Liu ZZ, Patzak A, and Georgieva R (2014): Novel Hemoglobin Particles - Promising New-Generation Hemoglobin-Based Oxygen Carriers; Artificial Organs 38(8):708-714. In brief, equal volumes of solution A consisting of 0.25 M of $MnCl_2$ and 10 mg/mL of HSA or Hb were rapidly mixed with solution B containing 0.25 M of $Na_2CO_3$ and 50 mg/mL of Odex in a beaker under vigorous stirring using a mechanical stirrer (model IKA RW 20 DZM equipped with a 35-mm diameter rotor) at room temperature. In this coprecipitation step, both proteins and inorganic $MnCO_3$ were precipitated together at the same time while the protein was crosslinked by Odex. Other biopolymers can be used as well in an oxidized form, examples are oxidized hydroxyethylstarch, oxidized hyaluronic acid, and oxidized alginate. All these polymers can be oxidized. However, their solubility in $Na_2CO_3$ is a prerequisite for their applicability.

After 30 seconds of co-precipitation, 5 mg/mL of HSA was added to the suspension and incubated for 5 min under stirring to result in the adsorption of HSA on the surface of particles. The added protein stops the crystallization (i.e. precipitication of the templates) by adsorption at the surface of the nanoparticles.

After incubation, the resulting suspension was centrifuged and the particles were washed three times with sterile 0.9% NaCl containing 0.2% HSA (centrifugation at 3000 x g for 3 min). Afterwards, the particles were proceeded to dissolution of the $MnCO_3$ template by 0.5 M EDTA/0.1M Glycine for 30 min and were reduced by 1% $NaBH_4$ dissolved in NaOH. Finally, the particles were centrifuged, washed three times and suspended in sterile 0.9% NaCl containing 2% HSA.

In order to characterize the obtained particles, the following parameters have been detected.

**Entrapment efficiency (EE%).** The amount of albumin in supernatant and APs was measured by Bradford Protein Assay Kit (Pierce Coomassie (Bradford) Protein Assay Kit - Thermo Fisher Scientific, USA). Hemoglobin concentrations of Hb solution and HbMP were determined by the cyano-methemoglobin method using a UV-vis spectrophotometer (Hitachi U2800, Hitachi High-Technologies Corporation). Protein entrapment efficiency of $MnCO_3$ was determined as the difference between the total amount ($P_t$) of applied albumin (Ab) or hemoglobin (Hb) and the Hb or Ab amount in the supernatant (Pf) after the co-precipitation and washing steps. Subsequently, the EE% was calculated according to the following equation: EE% = (Pt - Pf)$\times$ 100%/$P_t$.

**The volume concentration.** To detect the volume concentration, the particle suspension was processed similarly to the detection of hematocrit in whole blood. The particle suspension was filled into hematocrit capillary tubes and sealed with wax. Subsequently, the filled capillaries were centrifuged in a hematocrit centrifuge (Hettich Mikro 22R) at 20,000g for 10 min. The volume concentration values were then be read directly on a percentage reader.

**Hemoglobin content in HbMPs.** HbMP was mixed with pronase to achieve the mixture with final concentration of 2% (v/v) HbMP suspension which contains 0.5 mg/ml pronase in individual sample. The pronase-HbMP suspension was then incubated for 30 min at 45°C. Afterwards, alkaline hematin detergent (AHD) reagent (50 mg/mL of Triton X-100 and 0.2 M of NaOH) was introduced (1:1 ratio with Pronase-HbMP mixture) for hemin conversion. The absorbance values were recorded and substituted into the linear regression equation of hemin calibration curve and then can be converted to hemoglobin concentrations by calculation.

**Hydrodynamic particle diameter and zeta-potential.** The hydrodynamic diameter and the zeta-potential of the APs and HbMPs were measured by dynamic light scattering using a Zetasizer nano ZS instrument (Malvern Instruments Ltd., Malvern, UK) in 0.9% NaCl solution. The particle size values obtained by dynamic light scattering correlate well with the particle size values as obtained by atomic force microscopy (see below).

**Scanning electron microscopy.** For scanning electron microscopy imaging, samples were prepared by applying a drop of particles suspension onto glass followed by drying overnight. After sputtering with gold, measurements were conducted at an operation voltage of 3 keV using a Gemini Leo 1550 instrument (Oberkochen, Germany) and ImageJ 1.44p software (Wayne Rasband, National Institute of Mental Health, Bethesda, MD, USA).

**Atomic force microscopy (AFM).** As electron microscopy measurements can only be made on dried particles the obtained size values can merely be used for their comparison. To obtain realistic size values, AFM both on dried and hydrated (dissolved) particles can be applied. These measurements also allow detecting a degree of swelling of the particles (*cf.* Ijad Kao, Yu Xiong, Axel Steffen, Kathrin Smuda, Lian Zhao, Radostina Georgieva, Hans Bäumler (2018). Preclinical in vitro safety investigations of submicron sized hemoglobin based oxygen carrier HBMP-700. Artificial Organs 42(5):549-559). AFM images were obtained using a Nanoscope III Multimode AFM (Digital Instrument Inc., Santa Barbara, CA) in taping mode. The images were recorded and processed by the Nanoscope software. The samples were prepared by applying a drop of diluted particle suspension (dilution 1:10000 with deionized water) onto a freshly cleaved mica substrate. The mica substrate was pre-treated with polyethylene imine Mw 25 kDa, 1 mM for 20 min to obtain a positively charged surface for sufficient retention of the particles during the measurements. After allowing the particles to attach the substrate was rinsed with deionized water and dried under a gentle stream of nitrogen. The particles were first scanned in the dry state, then a drop of deionized water was added and the particles were scanned again in the wet state. Micro-lithographed tips on silicon nitride ($Si_3N_4$) cantilevers with a spring constant of 42 Nm$^{-1}$ and resonance frequency of 300 kHz (Olympus Corporation, Tokyo, Japan) were used for the measurements in air. The scans in water were performed with cantilevers with spring constant 3 Nm$^{-1}$ and resonance frequency 75 kHz (Budget Sensors, Innovative Solutions, Bulgaria Ltd., Sofia, Bulgaria). The height of each particle in the dry and wet state was measured applying the Nanoscope software as the height difference between the support and the highest point of the particle surface profile.

**Particles deformability.** Particle compression and dilation behavior were measured using an automatic measuring system based on an analytic centrifuge with an integrated optoelectronic sensor (LumiFuge, LUM GmbH, Berlin, Germany). 10% Hematocrit of particles were placed into the cuvette. The centrifugal forces were applied to particles as cycles in order to observe the compression and relaxation of particles. First, beginning with a centrifugal force of 5g, the packing of particle was performed shortly. Then, an increasing of centrifugal force up to 2300g was applied for 4 hours.

Finally, centrifugal force was reduced to 5g again for 4 hours in order to observe the relaxation of particles.

**Determination of oxygen carrying capacity of HbMPs.** The hemoglobin solution was diluted into different concentrations (0.75-6 mg/mL). 1 mL of each concentration was filled into a 2-mL glass vial containing a magnetic stirrer. After an equilibration time of 10 min, a miniaturized optical needle type oxygen sensor (oxygen microsensor NTH-PSt7, PreSens - Precision Sensing GmbH, Germany), which was connected to a portable oxygen meter with data logging (Microx 4, PreSens - Precision Sensing GmbH, Germany), was inserted into the stirred sample. The concentration of dissolved oxygen was recorded at the starting point. Then, 50 $\mu$L of 10% potassium ferricyanide ($K_3[Fe(CN)_6]$) dissolved in water were added, and the change in concentration of dissolved oxygen was measured and recorded every second. After reaching a stable value, the measurement was stopped. An increase of dissolved oxygen concentration after adding ferricyanide is dependent on the hemoglobin concentration-manner. The change in $pO_2$ is determined by subtracting the $pO_2$ from the beginning and the end of the measurement. We assume that the released oxygen was previously bound to hemoglobin. A calibration curve demonstrates the ratio of hemoglobin concentration vs. $pO_2$ at measurement time point. HbMPs were performed as the procedure described to the estimation of hemoglobin content in the particles compared with a determined calibration curve.

For evaluation of the interaction of the particles with cells, a phagocytosis assay of particles in whole blood was performed. As the obtained particles had no autofluorescence, the quantitative determination of granulocyte phagocytic activity was carried out in healthy human heparinized whole blood *in vitro* using the indirect measurement method. White blood cells (WBCs) were pre-fed with particles by incubation of particles for 0, 10, 30, 60, 120 min at 37°C, to determine the phagocytic activity of granulocytes in a kinetic-manner. Additionally, *E. coli* (non-fluorescein labeled from Phagoburst kit provided by Glycotope-Biotechnology GmbH, Heidelberg, Germany) and phosphate buffered saline (PBS) were used as positive and negative control, respectively. After reaching each incubation time point, FITC-labeled *E. coli* (from Phagotest kit provided by Glycotope-Biotechnology GmbH, Heidelberg, Germany) were added into the reaction tube and incubated further for 10 mins at 37°C. Afterward, the fluorescence quencher was added and washing steps were performed. Then, RBCs were lysed and DNA were fixed by RBC lysing solution and DNA fixation reagent (Glycotope-Biotechnology GmbH, Heidelberg, Germany), correspondingly. Finally, leukocytes were analyzed by flow cytometry (BD FACSCanto II, BD Bioscience, USA). The phagocytic percentage of granulocyte was analyzed. The signal of FITC-labeled *E. coli,* which was phagocyted by granulocytes demonstrated an inverse proportion of phagocytic activity to particles.

The influence of APs and HbMPs on human platelets was investigated in platelet-rich plasma (PRP). Human whole blood anticoagulated by citrated was centrifuged at 150g for 15 minutes at 20-25 °C, and the PRP fraction was collected. The platelet amount was detected before the test using a hematology analyzer ABX Micros 60 (HORIBA Europe GmbH, Berlin, Germany). PRP was gently mixed with particles suspension at a ratio of particles:platelets = 10:1 or sterile physiological saline (negative control). Then, pre-incubated human platelets were activated with platelet agonists (0.5 mg/mL arachidonic acid (AA), 0.2 mg/mL collagen, and 0.01 mM epinephrine (Epi): möLab GmbH, Germany) to induce platelet activation and aggregation. The mixture was incubated at 37°C for 30 min while shaking. Samples were then fixed by adding 9X volume of 0.5% (vol/vol) formaldehyde in phosphate-buffered saline. Finally, the number of activated platelets in fixed samples were measured by using allophycocyanine (APC) labelled anti-human CD42b antibody and Alexa Fluor® 488 anti-human CD62P (P-Selectin) antibody (BioLegend, San Diego, USA) by flow cytometry (BD FACSCanto II) and analyzed by BD FACSDIVA software (BD Biosciences, USA). For the analysis, a quadrant was set in the dot plot of respective channels on non-stimulated platelets. The appearance of double-colored events in the upper right quadrant (Q2) was quantified as activated platelets.

The results were presented as means $\pm$ standard deviation (SD) or standard error of the mean (SEM). The data were compared using ANOVA-like test. GraphPad Prism 6 software (GraphPad, La Jolla, CA, USA) was employed for graphs and statistical analyses. P-value <0.05 was considered statistically significant.

Oxidized-dextran (Odex), the polymeric cross-linker, was successfully prepared following a well-known method where dextran is oxidized with sodium periodate. The periodate oxidation of dextran is a two-step reaction. First, the periodate oxidation of an $\alpha$-1,6-linked anhydroglucoside unit leads to the formation of an $\alpha$-hydroxy aldehyde group. In the second step, hydroxy aldehyde group can be further oxidized generating formic acid and a dialdehyde group. Partial oxidation of dextran with periodate converts some of its vicinal hydroxyl groups into aldehyde (R-CHO) for each glucose subunit.

We used dextran with molecular weight 40 and 70 kDa because they are available for blood flow enhancement and/or plasma volume expansion. The actual number of aldehyde groups determined by hydroxylamine hydrochloride titration method was constant at approximately 155 aldehyde groups per 100 glucose subunits for both (Table 1). Thus, the number of aldehyde substitutions in activated dextran was independent of the molecular weight of the dextran substrate. Our results are similar to previously reported data where the number of aldehyde substitutions in activated dextran was investigated for dextran substrates with 6, 40, and 110 kDa MW and was constant at nearly 165 aldehyde groups per 100 glucose subunits.

Therefore, Odex is a promising candidate as a highly effective macromolecular cross-linker. The carbonyl groups of the sugar open-chains are capable of reacting with the free -NH$_2$ groups on the protein; the relatively fast formation of Schiff base results in creating an open-porous cross-linked network.

Table 1. Aldehyde content per 100 glucose subunits in the oxidized dextrans

| Type of Dextran | Number of aldehyde functional groups per 100 glucose units, average ± S.D. (*n=3*) |
|---|---|
| Dextran 40 kDa (40T-Odex) | 157±9 |
| Dextran 70 kDa (70T-Odex) | 152±6 |

The described one-pot particle fabrication technique is based on the coprecipitation-crosslinking-dissolution (CCD) procedure, which exploits the ability of insoluble carbonates to capture proteins and other macromolecules that are present in the aqueous solution during precipitation according to a first step. In a second step the incorporated biomacromolecules are cross-linked by a chemical reaction, typically with glutaraldehyde, and in a third step the inorganic components are dissolved with EDTA.

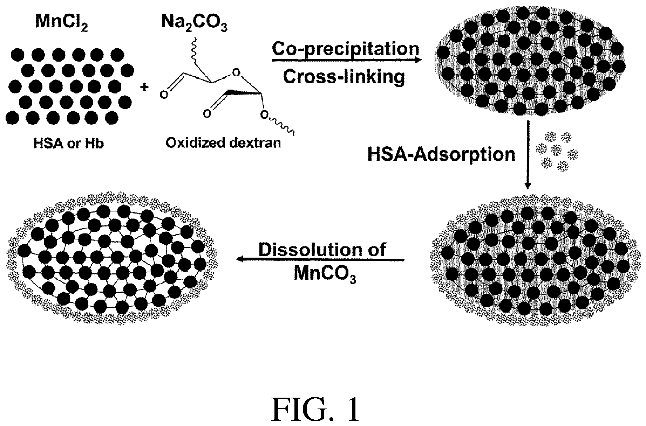

According to the presently suggested technique, the proteins are precipitated and cross-linked in one step because the macromolecular cross-linker (Odex) precipitates and is entrapped in the particles together with the proteins (Figure 1). In such a way one preparation step and several respective washing steps are reduced in our new procedure, which significantly reduces the preparation time.

The entrapment efficiency of HSA and Hb in the APs and HbMPs produced by the described one-pot technique was about 70%, typically between 50 % and 80 %, which had no significant difference to the efficiency of the CCD fabrication procedure reported by Xiong Y, Liu ZZ, Georgieva R, Smuda K, Steffen A, Sendeski M, et al. (2013) Nonvasoconstrictive hemoglobin particles as oxygen carriers. ACS Nano: 7(9):7454-61. Only a very small amount of albumin or hemoglobin has been found in the supernatant during the washing and dissolution steps.

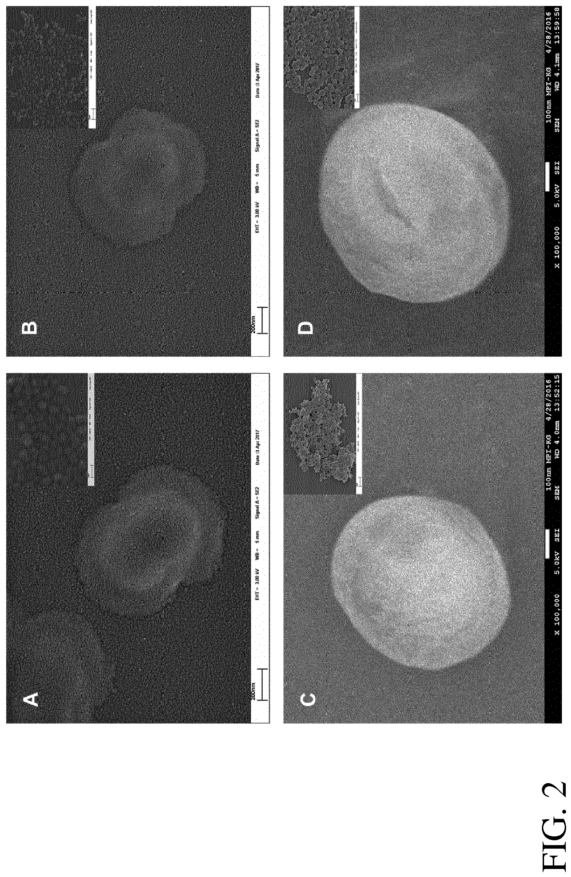

The hydrodynamic diameters of the albumin (APs) and hemoglobin particle (HbMPs) as measured by dynamic light scattering was around 850 nm. As revealed by scanning electron microscopy (*cf.* Fig. 2), Odex-crosslinked HbMPs appear to have a smooth surface and a peanut-like shape with shortest and longest diameter of 700 and 900 nm, respectively. Besides, autofluorescence of albumin particles (APs) and of HbMPs could not be observed by confocal laser scanning microscopy. Additionally, the APs and HbMPs suspended in 0.9% NaCl (conductivity 18 mS/cm) showed a negative zeta-potential of about -15 and -10 mV, respectively. To highlight, the measured physicochemical properties of albumin and hemoglobin particles either crosslinked by 40T- or 70T-Odex have no difference except the zeta-potential, which is related to the identity of the different proteins as shown in table 2.

**Table 2.** Characteristics of particles

| Particles type | Hydrodynamic diameter (nm) | Polydispersity index | $\zeta$-potential (mV) | Conductivity (mS/cm) |
|---|---|---|---|---|
| 40T-APs | 853±18 | 0.178±0.084 | -15±0.7 | 18 |
| 70T-APs | 826±33 | 0.197±0.057 | -15±0.5 | 18 |
| 40T-HbMPs | 861±20 | 0.140±0.076 | -10±0.4 | 18 |
| 70T-HbMPs | 869±18 | 0.219±0.038 | -10±0.2 | 18 |

**Table 3.** Hemoglobin content and functional hemoglobin in HbMPs

| Particles type | Entrapment efficiency [%] | Protein conc. in 20% Hct of particles [mg/mL] | Hb conc. in 20% Hct of particles [mg/mL] | Functional Hb in 20% Hct of particles [mg/mL] |
|---|---|---|---|---|
| 40T-APs | 67±9 | 28.0±4.5 | / | / |
| 70T-APs | 69±9 | 26.5±3.3 | / | / |
| 40T-HbMPs | 65±9 | / | 29.2±5.5 | 7.5±1.8 |
| 70T-HbMPs | 68±7 | / | 29.0±2.1 | 7.3±2.0 |
| In table 3 above "Hct" stands for hematocrit. | | | | |

The estimation of the functional hemoglobin content in the particles was investigated using the ferricyanide method. Ferricyanide (potassium hexacyanoferrate (III), $K_3[Fe(CN)_6]$) causes a conversion from oxyhemoglobin into methemoglobin and therefore previously hemoglobin-bound oxygen may be released into the surrounding media. The change in partial pressure of oxygen ($pO_2$) can be determined by the oxygen sensor. The result of 20% hematocrit (Hct) of 40T- and 70T-HbMPs showed that functional hemoglobin was $7.5 \pm 1.8$ and $7.3 \pm 2.0$ mg/mL, respectively.

Deformability of particles, including APs and HbMPs crosslinked with both 40T- and 70T-odex, were measured by an analytical centrifuge with an integrated optoelectric sensor system (LUMiFuge®, LUM GmbH, Berlin, Germany). In term of suspension stability measurements based on analysis of the light transmission profiles in horizontally arranged cuvettes in the purpose-designed rotor in dependence on time (STEP-Technology (Space- and Time-resolved Extinction Profiles). For the period of measurement, the samples exposed to the centrifugal forces deform (compress) and the light transmission profiles in the cuvettes shift depending on the applied force. The profiles are simultaneously registered along the cuvette by the STEP-Technology as a function of time and of the applied centrifugal force and analyzed using the software SEPVIEW 6 (LUM GmbH, 2013).

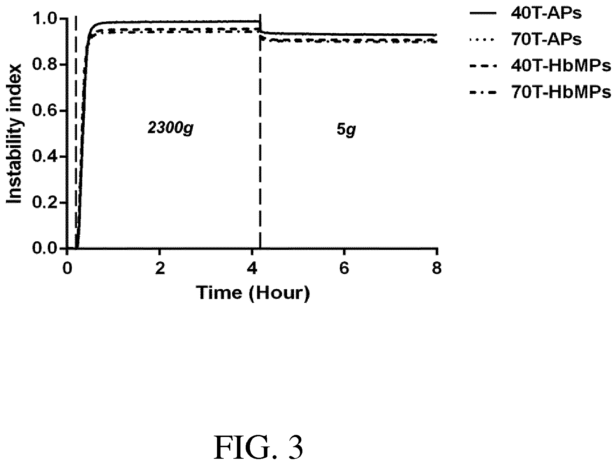

The results showed (cf. Fig. 3) that when particles exposed to force at 5g, rapidly packing was obtained but not change in particles volume occurred. After being exposed to centrifugal force at 2300g, particles were compressed and deformed which correspond with an increase of instability index (instability index=clarification as function of time/maximum clarification possible). However, after adjusting the centrifugation force to 5g again, a decreasing of instability index was observed which is the result of an increase of sediment volume. This phenomenon demonstrates the resilience, i.e. the elasticity of these particles.

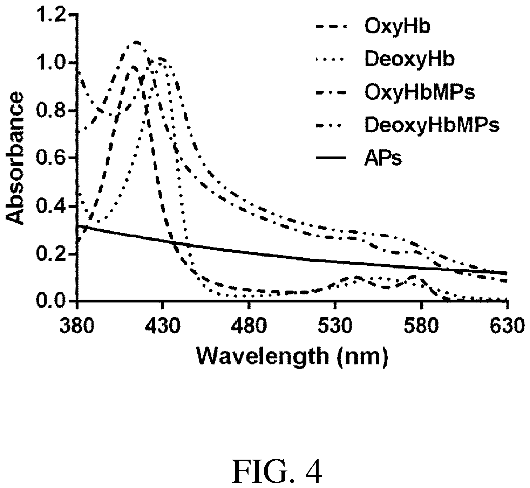

The UV-VIS spectrophotometric analysis exhibited the ability of HbMPs to bind and release oxygen in comparison to stroma-free hemoglobin as well as the albumin particle with the same preparation (cf. Fig. 4). Oxygenated HbMPs demonstrated three maximal peaks at 414, 542, and 576 nm which are corresponding to the characteristic absorption peaks of oxygenated Hb (OxyHb). After deoxygenation of the HbMPs using 1 mg/mL sodium dithionite (SDT), a red shift of the 414 nm peak (the Soret peak) to 432 nm was ensued. The peaks at 542 and 576 nm disappeared, and a peak at 556 nm was detected instead. The spectral behavior of HbMP showed a characteristic of oxygenated/deoxygenated Hb and confirmed the functionality of HbMPs to bind and release oxygen. Additionally, APs has no typical absorbance peak of hemoglobin solution or HbMPs. Beyond that, the spectra of particles (HbMPs and APs) was shifted toward higher absorption values over the whole wavelength interval resulting of particle's light scattering.

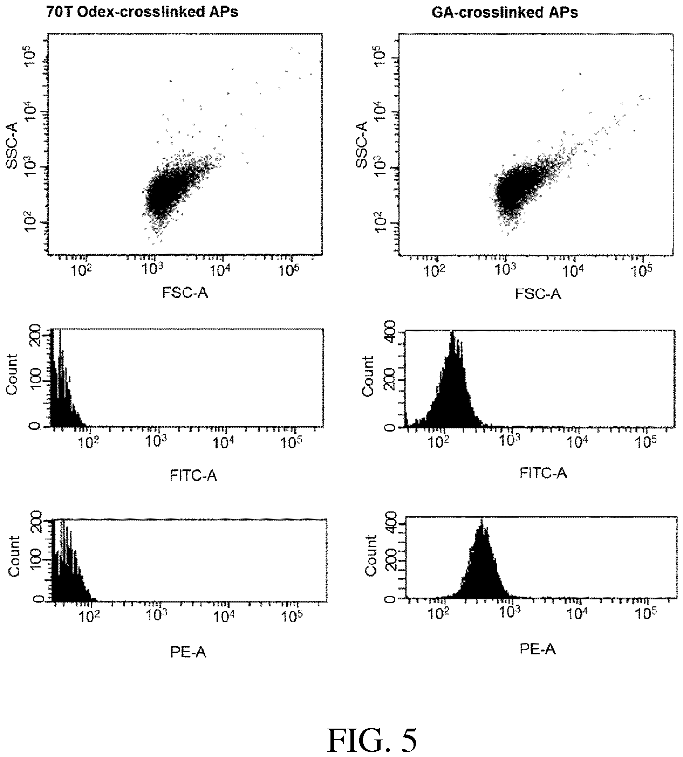

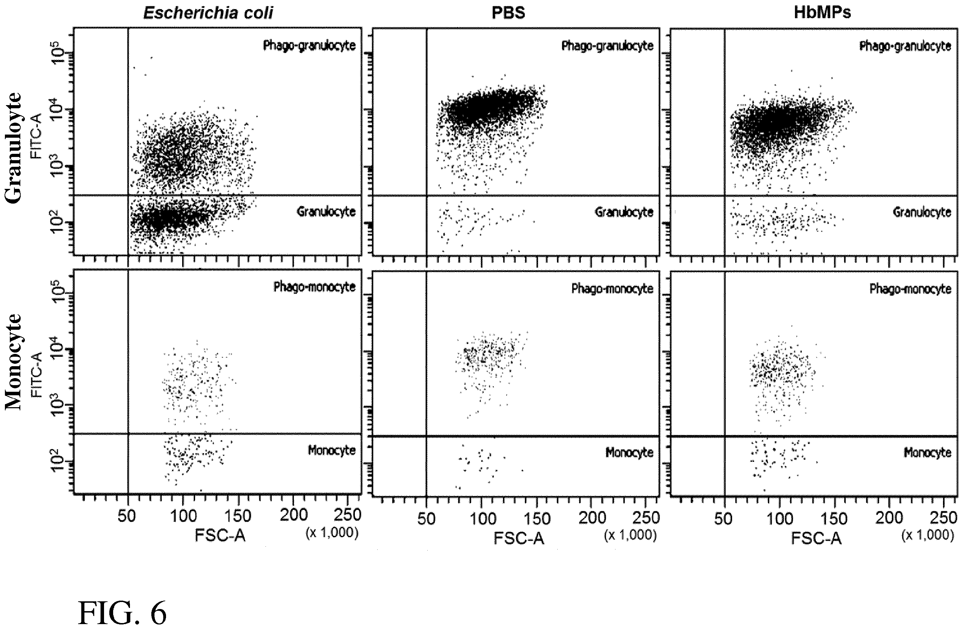

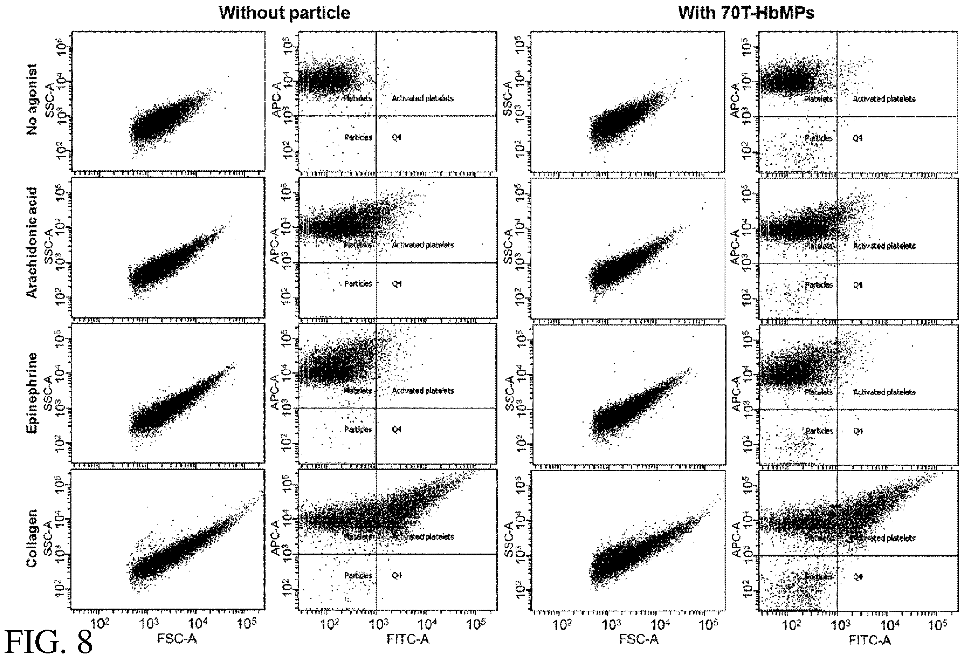

Additionally, the results from flow cytometry also yet demonstrated that particles produced by the suggested method using Odex as a crosslinker (Odex-APs) had no autofluorescence compared to particles which were crosslinked by glutaraldehyde (GA-APs) (cf. Fig. 5), which is visible in the fluorescence channels FITC-A and PE-A. Meanwhile, these results were correspondingly to other measurements concerning the dispersion of the particles. The side and forward scatter dot plot showed the impressive phenomenon of monodispersity of the obtained particles. For ease of statistical analysis of hydrodynamic diameters a fluorescence activated cell sorter (BD FACSCanto II, BD Bioscience, USA) was used. In Figs. 5 and 6 the abbreviation FSC stands for a forward scatter signal, in Figs. 5 and 8 the abbreviation SSC stands for a sideward scatter signal.

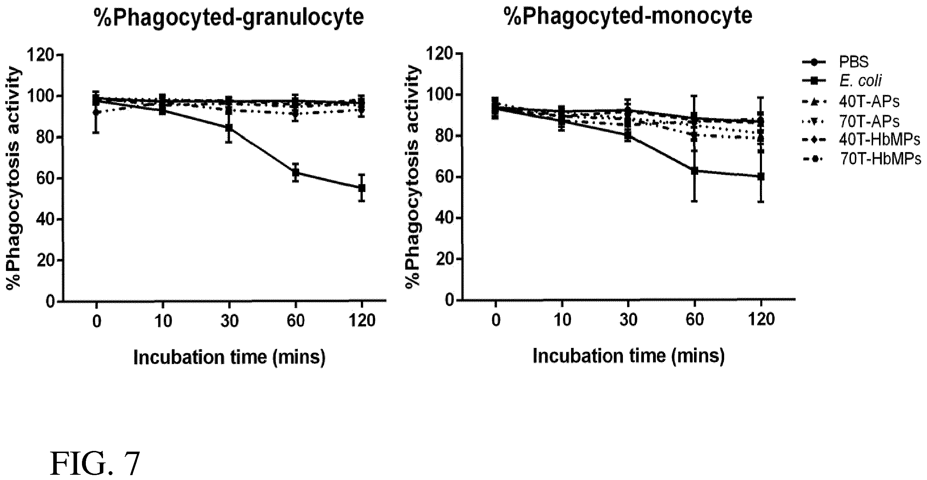

To evaluate the compatibility of our particles for medical usage, the indirect phagocytic assay was carried out in vitro (cf. Figs. 6 and 7). We modified the commercially available Phagotest kit which determines the percentage of phagocytes which ingest fluorescein-isothiocyanate (FITC) labelled opsonized E. coli bacteria and their activity (number of bacteria per cell) which allows the quantitative determination of leukocyte phagocytosis (uptake of bacteria). Therefore, particles which have autofluorescence can be used to determine the biocompatibility of the particles by this kit similarly as the FITC-labelled E. coli. In order to investigate the phagocytic function of human granulocytes and monocytes on non-fluorescein particles, phagocytes were pre-fed with APs, HbMPs, nonfluorescein labeled-E. coli as positive control and PBS as negative control individually for 10, 30, 60 and 120 minutes at 37°C to allow saturated phagocytosis of granulocytes. Then, FITC-labeled E. coli, which was used as an indicator, was added to pre-feed WBCs and incubated for 10 mins at 37°C. Hereby, empty-WBCs are able to phagocytose indicator bacteria while phagocytosed WBCs are not able to engulf more in this step. The phagocytic activity of granulocyte was quantified by flow cytometry.

The phagocytic activity results of granulocytes and monocytes showed that there was no difference between particles and PBS, which was a negative control. In contrast, the positive control demonstrated a gradually decrease of percentage of phagocytic activity in time-dependent manner.

Additionally, to evaluate whether the APs and HbMPs as generated according to the suggested one-pot co-precipitation technique alter the hemostasis system, the influence of APs and HbMPs on platelet activation was measured. Each APs and HbMPs were pre-incubated with platelet-rich-plasma (PRP) in a particle:platelet ratio 10:1 for 30 min at 37°C under gentle agitation. After which agonists including arachidonic acid (AA), collagen and epinephrine (Epi) were applied to induce platelet activation. The activated platelets were quantified using APC anti-human CD42b (GPIb$\alpha$) antibody and Alexa Fluor® 488 anti-human CD62P (P-Selectin) antibody (BioLegend, San Diego, USA) by flow cytometer. Therefore, difference stained pattern could distinguish between inactivated platelet and activated platelet. On one hand, single stained population with CD42b was defined as inactivated platelet. On the other hand, the double-stained with anti-CD42ba and anti-CD62P population was defined as activated platelet. The number of pre-activated platelets (PRP without particles or agonists) were used to subtract the pre-activation background. The results (*cf.* Fig. 8) showed that the number of activated platelets where platelets were incubated with APs or HbMP had no significant difference compared to the control sample which was incubated with sterile physiological saline (negative control).

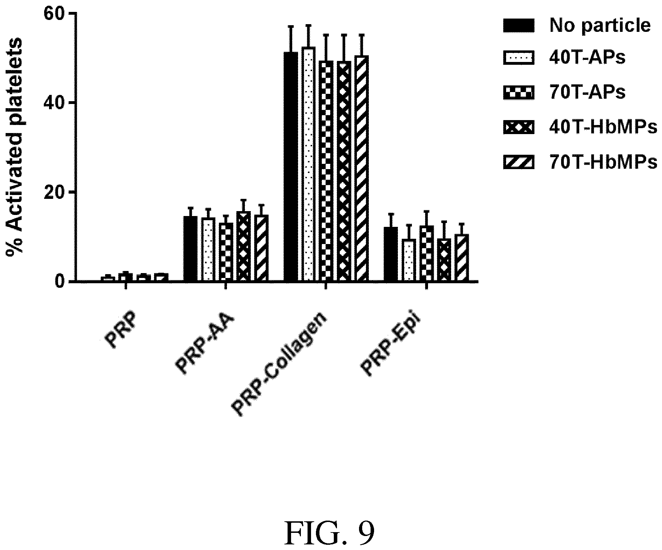

Additionally, platelet activation induced by platelet agonists including arachidonic acid, collagen and epinephrine of pre-incubated PRP with particles was comparable to the control sample which was pre-incubated with physiological saline. Simultaneously, upon the stimulation of agonists on platelets, aggregation of platelets was also observed as the shift of forward scatter/side scatter positioning compared with negative control. As evident, APs and HbMP do not influence the activation and aggregation of platelets (*cf.* Fig. 9).

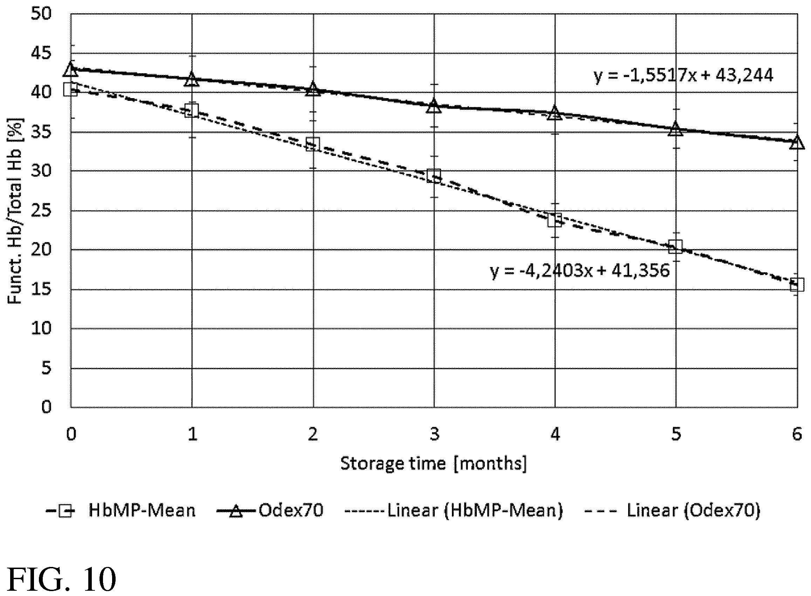

As illustrated by Fig. 10, the suggested cross-linker Odex (oxidized dextran 70 kDa) allows for a remarkable preservation of biological activity of the cross-linked protein in the obtained particles in comparison to cross-linking by glutaraldehyde. In the shown examples two batches (each N=3) of hemoglobin particles, one prepared with the cross-linker Odex and the other one prepared with the cross-linker glutaraldehyde, have been stored at a concentration of 20 % hematocrit over a period of 6 months. Whereas the initial concentration of active Hb in the particles is nearly identical, the decline in activity is remarkably slower (-1.551 vs. -4.240093) for particles cross-linked with Odex when compared with GA cross-linked hemoglobin particles (HbMP).

As shown by the example above, we have successfully established a novel, simple, efficient, and cost-effective method for biopolymer particle fabrication modified from CCD technique using Odex as a biocompatible cross-linker. Monodisperse protein particles including albumin particles (APs) and hemoglobin particles (HbMPs) could be prepared by coprecipitation of albumin or hemoglobin with the $MnCO_3$ template and crosslinking with Odex were taking place at one-step. After removing $MnCO_3$ template with EDTA, the resulting APs and HbMPs showed a negative charge and a size about 850 nm with homogeneous morphology. HbMPs could maintain their ability to be oxygenated/deoxygenated. Moreover, APs and HbMPs also demonstrated their low immunogenicity and had no influence on agonist induced-platelet activation. These results suggest that Odex can be used as an alternative cross-linker for obtaining protein particles. However, other carbohydrates, once oxidized, can be used as cross-linker as well. Examples are: oxidized HAES (hydroxyethyl starch), oxidized hyaluronic acid and oxidized alginate.

Other biomolecules which can be cross-linked by an oxidized dextran or by one of the other oxidized carbohydrates can be selected from the group consisting of lectins (e.g. ConA), Insulin and thyroxine.

To summarize, a method of manufacturing biopolymer particles is suggested, the method comprising: fabricating an aqueous suspension of precipitate particles by mixing together a first salt, a second salt, a biopolymer, and an oxidized carbohydrate, the carbohydrate having a molecular weight of 10 kDa or more, wherein the first and the second salt are selected such that the anion of the first salt and the cation of the second salt together form precipitate particles, wherein upon mixing the biopolymer and the carbohydrate are incorporated in the precipitate particles and cross-link with each other; washing the suspension of precipitate particles; dissolving the precipitate particles by adding a chelator and releasing the cross-linked biopolymer and the carbohydrate as biopolymer particles.

**Claims**

1. A method of manufacturing biopolymer particles, the method comprising:

   - fabricating an aqueous suspension of precipitate particles by mixing together a first salt, a second salt, a biopolymer, and an oxidized carbohydrate having a molecular weight of 10 kDa or more,
   wherein the oxidized carbohydrate is selected from an oxidized dextran, an oxidized hydroxyethyl starch, an oxidized hyaluronic acid, an oxidized alginate, an oxidized chitosan, an oxidized chondroitin, an oxidized pectin, and an oxidized cellulose,
   wherein the biopolymer is selected from the group consisting of: proteins originated from plants, algae or animals, amino-carbohydrates originated from plants, algae or animals, peptides originated from plants, algae or animals, and nucleic acids originated from plants, algae or animals,
   wherein the first and the second salt have anions and cations of inorganic nature, wherein the first and the second salt are selected such that the anion of the first salt and the cation of the second salt together form a template structure of the precipitate particles, wherein upon mixing the biopolymer and the carbohydrate are incorporated in the template structure of the precipitate particles and cross-link with each other;
   - washing the suspension of precipitate particles; and
   - dissolving the template structure of the precipitate particles by adding a chelator and releasing the cross-linked biopolymer and the carbohydrate as biopolymer particles.

2. The method according to claim 1, wherein the chelator is selected from the group consisting of: diethylenetriamine pentaacetic acid, ethylenediamine tetracetic acid, N-hydroxyethylethylenediamine triacetic acid, nitrilo triacetic acid, and salts thereof.

3. The method according to claim 1 or 2, wherein the first salt and the second salt are provided in separate solutions, and wherein the biopolymer and the oxidized carbohydrate are provided in separate solutions.

4. The method according to any of the claims 1 to 3, wherein the oxidized carbohydrate comprises an oxidized dextran.

5. The method according to any of claims 1 to 4, wherein the biopolymer particles have a D90 diameter of 10 μm or less, particularly of 5 μm or less, more particularly of 2 μm or less.

6. The method according to any of the previous claims, further comprising fractionizing the biopolymer particles with respect to size to separate particles having a size of 500 nm or less from the remaining particles.

7. The method according to any of claims 4 to 6, wherein the dextran has a molecular weight of between 10 kDa and 2 MDa, in particular a molecular weight of between 20 kDa and 1 MDa, more particularly a molecular weight of between 30 kDa and 400 kDa, and even more particularly a molecular weight of between 40 kDa and 100 kDa.

8. A biopolymer particle comprising a three-dimensional network structure extending through the volume of the biopolymer particle, wherein the three-dimensional network structure is formed at least by a biopolymer, and a carbohydrate cross-linking the biopolymer, wherein
the carbohydrate has a molecular weight of 10 kDa or more, and the carbohydrate is selected from dextran, hydroxyethyl starch, hyaluronic acid, alginate, chitosan, chondroitin, pectin, and cellulose,
wherein the biopolymer is selected from the group consisting of: proteins originated from plants, algae or animals, amino-carbohydrates originated from plants, algae or animals, peptides originated from plants, algae or animals, and nucleic acids originated from plants, algae or animals, and
wherein the three-dimensional network has a molecular weight of at least 1 MDa.

9. The biopolymer particle according to claim 8, wherein the content of the biopolymer in the 3-dimensional network is from about 40% through about 85% (w/w), and a carbohydrate content of the biopolymer particle comprises from about 15% through about 60% (w/w), based on the total weight of the 3-dimensional network.

10. The biopolymer particle according to any of claims 8 to 9, wherein the protein comprises a heme protein, in particular hemoglobin or myoglobin, and the biopolymer particle is able to bind and to release, depending on a partial gas pressure, both oxygen and carbon dioxide.

11. The biopolymer particle according to any of claims 8 to 10, wherein the biopolymer particle is suspended in a physiological solution, in particular in a 0.9 % aqueous NaCl solution, in a Ringer's salt solution, or in a serum.

12. The biopolymer particle according to any of claims 8 to 11, further comprising a releasable drug, wherein the releasable drug is bound to the 3-dimensional network.

13. The biopolymer particle according to any of claims 8 to 12, wherein the particle is surface-functionalized.

14. A use of an oxidized carbohydrate having a molecular weight of at least 10 kDa to form biopolymer particles, wherein the biopolymer particles comprises a three-dimensional network structure extending through the volume of the particle, wherein the three-dimensional network structure is formed at least by a biopolymer and a carbohydrate, the carbohydrate cross-linking the biopolymer, wherein the carbohydrate is selected from a dextran, a hydroxyethyl starch, a hyaluronic acid, an alginate, a chitosan, a chondroitin, a pectin, and a cellulose, wherein the carbohydrate has a molecular weight of 10 kDa or more and wherein the oxidized carbohydrate is selected from an oxidized dextran, an oxidized hydroxyethyl starch, an oxidized hyaluronic acid, an oxidized alginate, an oxidized chitosan, an oxidized chondroitin, an oxidized pectin, and an oxidized cellulose, and wherein the three-dimensional network has a molecular weight of at least 1 MDa, for instance of at least 5 MDa, specifically of at least 10 MDa.

**Patentansprüche**

1. Verfahren zur Herstellung von Biopolymer-Partikeln, umfassend:

- Herstellen einer wässrigen Suspension von Präzipitat-Partikeln durch Zusammenmischen eines ersten Salzes, eines zweiten Salzes, eines Biopolymers, und eines oxidierten Kohlenhydrats das ein Molekulargewicht von 10 kDa oder mehr hat,
wobei das oxidierte Kohlenhydrat ausgewählt ist aus einem oxidierten Dextran, einer oxidierten Hydroxyethyl-Stärke, einer oxidierten Hyaluronsäure, einem oxidierten Alginat, einem oxidierten Chitosan, einem oxidierten Chondroitin, einem oxidierten Pektin, und einer oxidierten Zellulose,
wobei das Biopolymer ausgewählt ist aus der Gruppe bestehend aus: Proteine aus Pflanzen, Algen oder Tieren, Amino-Kohlenhydrate aus Pflanzen, Algen oder Tieren, Peptide aus Pflanzen, Algen oder Tieren, und Nukleinsäuren aus Pflanzen, Algen oder Tieren,
wobei das erste und das zweite Salz Anionen und Kationen anorganischer Natur haben, wobei das erste und das zweite Salz so ausgewählt sind, dass das Anion des ersten Salzes und das Kation des zweiten Salzes gemeinsam eine Templat-Struktur der Präzipitat-Partikel ausbilden, wobei beim Zusammenmischen das Biopolymer und das Kohlenhydrat in die Templat-Struktur der Präzipitat-Partikel inkorporiert werden und miteinander vernetzen;
- Waschen der Suspension von Präzipitat-Partikeln; und
- Auflösen der Templat-Struktur der Präzipitat-Partikel durch Zusetzen eines Chelators und Freisetzen des vernetzten Biopolymers und des Kohlenhydrats als Biopolymer-Partikeln.

2. Verfahren nach Anspruch 1, wobei der Chelator ausgewählt ist aus der Gruppe, bestehend aus: Diethylentriaminpentaessigsäure, Ethylendiamintetraessigsäure, N-Hydroxyethylethylendiamintriessigsäure, Nitrilotriessigsäure, und deren Salzen.

3. Verfahren nach Anspruch 1 oder 2, wobei das erste Salz und das zweite Salz in getrennten Lösungen bereitgestellt werden, und wobei das Biopolymer und das oxidierte Kohlenhydrat in getrennten Lösungen bereitgestellt werden.

4. Verfahren nach einem der Ansprüche 1 bis 3, wobei das oxidierte Kohlenhydrat ein oxidiertes Dextran umfasst.

5. Verfahren nach einem der Ansprüche 1 bis 4, wobei die Biopolymer-Partikeln einen D90 Durchmesser von 10 $\mu$m oder weniger, insbesondere von 5 $\mu$m oder weniger, ganz besonders von 2 $\mu$m oder weniger haben.

6. Verfahren nach einem der vorherigen Ansprüche, weiter umfassend Fraktionieren der Biopolymer-Partikeln in Hinsicht auf Größe, um Partikeln, die eine Größe von 500 nm oder geringer haben, von den verbleibenden Partikeln abzutrennen.

7. Verfahren nach einem der Ansprüche 4 bis 6, wobei das Dextran ein Molekulargewicht hat zwischen 10 kDa und 2 MDa, insbesondere ein Molekulargewicht zwischen 20 kDa und 1 MDa, weiter besonders ein Molekulargewicht zwischen 30 kDa und 400 kDa, und ganz besonders ein Molekulargewicht zwischen 40 kDa und 100 kDa.

8. Biopolymer-Partikel umfassend eine drei-dimensionale Netzwerkstruktur die sich durch das Volumen des Biopolymer-Partikels erstreckt, wobei die drei-dimensionale Netzwerkstruktur wenigstens gebildet ist durch ein Biopolymer und ein das Biopolymer vernetzendes Kohlenhydrat, wobei
das Kohlenhydrat ein Molekulargewicht von 10 kDa oder mehr hat, und das Kohlenhydrat ausgewählt ist aus einem Dextran, einer Hydroxyethyl-Stärke, einer Hyaluronsäure, einem Alginat, einem Chitosan, einem Chondroitin, einem Pektin, und einer Zellulose,
wobei das Biopolymer ausgewählt ist aus der Gruppe, bestehend aus: Proteine aus Pflanzen, Algen oder Tieren, Amino-Kohlenhydrate aus Pflanzen, Algen oder Tieren, Peptide aus Pflanzen, Algen oder Tieren, und Nukleinsäuren aus Pflanzen, Algen oder Tieren,
wobei das drei-dimensionale Netzwerk ein Molekulargewicht hat von zumindest 1 MDa.

9. Biopolymer-Partikel nach Anspruch 8, wobei der Gehalt des Biopolymers in dem 3-dimensionalen Netzwerk von 40% bis etwa 85% (w/w) beträgt, und ein Kohlenhydrat-Gehalt des Biopolymer-Partikels von etwa 15% bis etwa 60% (w/w), basierend auf dem Gesamtgewicht des 3-dimensionalen Netzwerks, umfasst.

10. Biopolymer-Partikel nach einem der Ansprüche 8 oder 9, wobei das Protein ein Hämoprotein, insbesondere Hämoglobin oder Myoglobin umfasst, und das Biopolymer-Partikel geeignet ist, sowohl Sauerstoff als auch Kohlendioxid, abhängig von einem Partialgasdruck, zu binden und freizusetzen.

11. Biopolymer-Partikel nach einem der Ansprüche 8 bis 10, wobei das Biopolymer-Partikel suspendiert ist in einer physiologischen Lösung, insbesondere in einer 0.9 % wässrigen NaCl Lösung, in einer Ringerlösung, oder in einem Serum.

**12.** Biopolymer-Partikel nach einem der Ansprüche 8 bis 11, weiter umfassend ein freisetzbares Medikament, wobei das freisetzbare Medikament an das 3-dimensionale Netzwerk gebunden ist.

**13.** Biopolymer-Partikel nach einem der Ansprüche 8 bis 12, wobei das Partikel oberflächenmodifiziert ist.

**14.** Verwendung eines oxidierten Kohlenhydrats, das ein Molekulargewicht von wenigstens 10 kDa hat, um Biopolymer-Partikel auszubilden, wobei die Biopolymer-Partikel eine sich durch das Volumen des Partikels erstreckende drei-dimensionale Netzwerkstruktur umfassen, wobei die drei-dimensionale Netzwerktruktur gebildet ist zumindest durch ein Biopolymer und ein Kohlenhydrat, wobei das Kohlenhydrat das Biopolymer vernetzt, wobei das Kohlenhydrat ausgewählt ist aus einem Dextran, einer Hydroxyethyl-Stärke, einer Hyaluronsäure, einem Alginat, einem Chitosan, einem Chondroitin, einem Pektin, und einer Zellulose, wobei das Kohlenhydrat ein Molekulargewicht von 10 kDa oder mehr hat und wobei das oxidierte Kohlenhydrat ausgewählt ist aus einem oxidierten Dextran, einer oxidierten Hydroxyethyl-Stärke, einer oxidierten Hyaluronsäure, einem oxidierten Alginat, einem oxidierten Chitosan, einem oxidierten Chondroitin, einem oxidierten Pektin, und einer oxidierten Zellulose, und wobei das drei-dimensionale Netzwerk ein Molekulargewicht von zumindest 1 MDa hat, beispielsweise von zumindest 5 MDa, spezifisch von zumindest 10 MDa.

## Revendications

**1.** Procédé de fabrication de particules de biopolymère, le procédé comprenant :

- la fabrication d'une suspension aqueuse de particules de précipité en mélangeant ensemble un premier sel, un second sel, un biopolymère, et un hydrate de carbone oxydé ayant une masse moléculaire de 10 kDa ou supérieure,
dans lequel l'hydrate de carbone oxydé est choisi parmi un dextrane oxydé, un hydroxyéthylamidon oxydé, un acide hyaluronique oxydé, un alginate oxydé, un chitosane oxydé, une chondroïtine oxydée, une pectine oxydée et une cellulose oxydée,
dans lequel le biopolymère est choisi dans le groupe consistant en : protéines provenant de plantes, algues ou animaux, amino-hydrates de carbone provenant de plantes, algues ou animaux, peptides provenant de plantes, algues ou animaux, et acides nucléiques provenant de plantes, algues ou animaux,
dans lequel le premier et le second sel présentent des anions et cations de nature inorganique, dans lequel le premier et le second sel sont choisis de sorte que l'anion du premier sel et le cation du second sel forment ensemble une structure de matrice des particules de précipité, dans lequel lors du mélange le biopolymère et l'hydrate de carbone sont incorporés dans la structure de matrice des particules de précipité et réticulent l'un avec l'autre ;
- le lavage de la suspension des particules de précipité ; et
- la dissolution de la structure de matrice des particules de précipité par addition d'un chélateur et libération du biopolymère réticulé et de l'hydrate de carbone comme particules de biopolymère.

**2.** Procédé selon la revendication 1, dans lequel le chélateur est choisi dans le groupe consistant en : acide diéthylènetriamine pentaacétique, acide éthylènediamine tétraacétique, acide N-hydroxyéthylènediamine triacétique, acide nitrilo triacétique, et sels de ceux-ci.

**3.** Procédé selon la revendication 1 ou 2, dans lequel le premier sel et le second sel sont fournis dans des solutions séparées, et dans lequel le biopolymère et l'hydrate de carbone oxydé sont fournis dans des solutions séparées.

**4.** Procédé selon l'une quelconque des revendications 1 à 3, dans lequel l'hydrate de carbone oxydé comprend un dextrane oxydé.

**5.** Procédé selon l'une quelconque des revendications 1 à 4, dans lequel les particules de biopolymère présentent un diamètre D90 de 10 $\mu$m ou inférieur, particulièrement de 5 $\mu$m ou inférieur, encore mieux de 2 $\mu$m ou inférieur.

**6.** Procédé selon l'une quelconque des revendications précédentes, comprenant de plus le fractionnement des particules de biopolymère par rapport à la taille pour séparer des particules ayant une taille de 500 nm ou inférieure des particules restantes.

7. Procédé selon l'une quelconque des revendications 4 à 6, dans lequel le dextrane présente une masse moléculaire de 10 kDa à 2 MDa, en particulier une masse moléculaire de 20 kDa à 1 MDa, encore mieux une masse moléculaire de 30 kDa à 400 kDa, et bien mieux encore une masse moléculaire de 40 kDa à 100 kDa.

8. Particule de biopolymère comprenant une structure de réseau tridimensionnel s'étendant à travers le volume de la particule de biopolymère, dans laquelle la structure de réseau tridimensionnel est formée d'au moins un biopolymère, et d'un hydrate de carbone réticulant le biopolymère, dans laquelle
l'hydrate de carbone présente une masse moléculaire de 10 kDa ou supérieure, et l'hydrate de carbone est choisi parmi un dextrane, hydroxyéthylamidon, acide hyaluronique, alginate, chitosane, une chondroïtine, pectine et cellulose,
dans laquelle le biopolymère est choisi dans le groupe consistant en : protéines provenant de plantes, algues ou animaux, amino-hydrates de carbone provenant de plantes, algues ou animaux, peptides provenant de plantes, algues ou animaux, et acides nucléiques provenant de plantes, algues ou animaux, et
dans laquelle le réseau tridimensionnel présente une masse moléculaire d'au moins 1 MDa.

9. Particule de biopolymère selon la revendication 8, dans laquelle la teneur du biopolymère dans le réseau 3-dimensionnel est d'environ 40 % à environ 85 % (masse/masse), et une teneur en hydrate de carbone de la particule de biopolymère est d'environ 15 % à environ 60 % (masse/masse), rapporté à la masse totale du réseau 3-dimensionnel.

10. Particule de biopolymère selon l'une quelconque des revendications 8 à 9, dans laquelle la protéine comprend une protéine à hème, en particulier hémoglobine ou myoglobine, et la particule de biopolymère est apte à se lier et à se détacher, selon une pression partielle de gaz, à la fois d'oxygène et de dioxyde de carbone.

11. Particule de biopolymère selon l'une quelconque des revendications 8 à 10, dans laquelle la particule de biopolymère est mise en suspension dans une solution physiologique, en particulier dans une solution aqueuse de NaCl à 0,9 %, dans une solution de sel de Ringer, ou dans un sérum.

12. Particule de biopolymère selon l'une quelconque des revendications 8 à 11, comprenant de plus un médicament libérable, dans laquelle le médicament libérable est lié au réseau 3-dimensionnel.

13. Particule de biopolymère selon l'une quelconque des revendications 8 à 12, dans laquelle la particule est fonctionnalisée en surface.

14. Utilisation d'un hydrate de carbone oxydé ayant une masse moléculaire d'au moins 10 kDa pour former des particules de biopolymère, dans laquelle les particules de biopolymère comprennent une structure de réseau tridimensionnel s'étendant à travers le volume de la particule, dans laquelle la structure de réseau tridimensionnel est formée d'au moins un biopolymère et d'un hydrate de carbone, l'hydrate de carbone réticulant le biopolymère, dans laquelle l'hydrate de carbone est choisi parmi un dextrane, un hydroxyéthylamidon, un acide hyaluronique, un alginate, un chitosane, une chondroïtine, une pectine, et une cellulose, dans laquelle l'hydrate de carbone présente une masse moléculaire de 10 kDa ou supérieure et dans laquelle l'hydrate de carbone oxydé est choisi parmi un dextrane oxydé, un hydroxyéthylamidon oxydé, un acide hyaluronique oxydé, un alginate oxydé, un chitosane oxydé, une chondroïtine oxydée, une pectine oxydée, et une cellulose oxydée, et dans laquelle le réseau tridimensionnel présente une masse moléculaire d'au moins 1 MDa, par exemple d'au moins 5 MDa, spécifiquement d'au moins 10 MDa.

FIG. 1

FIG. 2

FIG. 3

FIG. 4

**FIG. 5**

FIG. 6

FIG. 7

FIG. 8

FIG. 9

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **D.V. VOLODKIN ; S. SCHMIDT ; P. FERNANDES ; N.I. LARIONOVA ; G.B. SUKHORUKOV ; C. DUSCHL ; H. MÖHWALD ; R. VON KLITZING.** *Advanced Functional Materials,* 2012, vol. 22, 1914-1922
- **YU XIONG ; RADOSTINA GEORGIEVA ; AXEL STEFFEN ; KATHRIN SMUDA ; HANS BÄUMLER.** *Journal of Colloid and Interface Science,* 2018, vol. 514, 156-164
- **YU XIONG ; AXEL STEFFEN ; KRISTIN ANDREAS ; SUSANNE MÜLLER ; NADINE STERNBERG ; RADOSTINA GEORGIEVA ; HANS BÄUMLER.** *Biomacromolecules,* 2012, vol. 13, 3292-3300
- **XIONG, Y. ; GEORGIEVA, R. ; STEFFEN, A. ; SMUDA, K. ; BÄUMLER, H.** Structure and properties of hybrid biopolymer particles fabricated by co-precipitation cross-linking dissolution procedure. *Journal of Colloid and Interface Science,* 2018, vol. 514, 156-164

- **HUANG YR ; HUNG YC ; HSU SY ; HUANG YW ; HWANG DF.** Application of electrolyzed water in the food industry. *Food Control,* 2008, vol. 19, 329-345
- **ZEIGER E ; GOLLAPUDI B ; SPENCER P.** Genetic toxicity and carcinogenicity studies of glutaraldehyde - a review. *Mutation Research/Reviews in Mutation Research,* 2005, vol. 589 (2), 136-151
- **BÄUMLER H ; XIONG Y ; LIU ZZ ; PATZAK A ; GEORGIEVA R.** Novel Hemoglobin Particles - Promising New-Generation Hemoglobin-Based Oxygen Carriers. *Artificial Organs,* 2014, vol. 38 (8), 708-714
- **IJAD KAO ; YU XIONG ; AXEL STEFFEN ; KATHRIN SMUDA ; LIAN ZHAO ; RADOSTINA GEORGIEVA ; HANS BÄUMLER.** Preclinical in vitro safety investigations of submicron sized hemoglobin based oxygen carrier HBMP-700. *Artificial Organs,* 2018, vol. 42 (5), 549-559
- **XIONG Y ; LIU ZZ ; GEORGIEVA R ; SMUDA K ; STEFFEN A ; SENDESKI M et al.** Nonvasoconstrictive hemoglobin particles as oxygen carriers. *ACS Nano,* 2013, vol. 7 (9), 7454-61